# FASCICULE DE BREVET EUROPEEN

(11) **EP 3 863 497 B1**
(45) Date de publication et mention de la délivrance du brevet: **28.12.2022**
(21) Numéro de dépôt: 19773466.8
(22) Date de dépôt: 27.09.2019
(51) Int. Cl.: A61B 1/00, G06F 13/38, G06F 13/42, A61B 3/00

(54) **SYSTEME DE COMMANDE D'AU MOINS UN APPAREIL D'EXAMEN DE VUE ET PROCEDE ASSOCIE**
SYSTEM ZUR STEUERUNG MINDESTENS EINER SEHTESTVORRICHTUNG UND ZUGEHÖRIGES VERFAHREN
SYSTEM FOR CONTROLLING AT LEAST ONE EYESIGHT TESTING DEVICE AND ASSOCIATED METHOD

(30) Priorité: 10.10.2018 FR 1859396
(43) Date de publication de la demande: 18.08.2021
(73) Titulaire: Siview SAS, 91460 Marcoussis (FR)
(72) Inventeur: GUILLEMAIN, Nathalie, 95320 Saint Leu la Forêt (FR)
(74) Mandataire: Cabinet Camus Lebkiri
(86) Numéro de dépôt international: PCT/EP2019/076255
(87) Numéro de publication internationale: WO 2020/074279

(56) Documents cités:
- US-A1- 2009 193 435
- US-A1- 2015 295 764
- US-B2- 7 730 242

## Description

### DOMAINE TECHNIQUE

La présente invention est relative à un système de commande d'au moins un appareil d'examen de vue ; un boîtier modulaire du système de commande ; un boîtier de pilotage du système de commande ; et un procédé de commande d'au moins un appareil d'examen de vue au moyen du système de commande.

### ÉTAT DE LA TECHNIQUE

Plusieurs appareils sont susceptibles d'intervenir lors d'un examen de vue, parmi lesquels :
- de manière facultative mais fréquente :
   o un autoréfractomètre pour une mesure objective des caractéristiques de l'œil,
   o un frontofocomètre pour mesurer les caractéristiques optiques des verres de lunettes du patient s'il en a déjà,
   o un ordinateur comportant un logiciel de gestion des patients ;
- de manière systématique sauf à utiliser une paire de lunettes d'essai ou une tête de réfracteur manuelle :
   o une tête de réfracteur automatique afin de placer des verres devant un œil ou les yeux d'un patient et une console de commande de la tête de réfracteur automatique afin de piloter la mise en place des verres,
   o ainsi qu'un écran d'optotype ou un projecteur d'optotype afin d'afficher les tests à lire.

Ces appareils interviennent de manière complémentaire, chacun lors d'une étape de l'examen de vue : les données fournies en sortie par l'un vont être utilisées en entrée par un autre. Par exemple, l'autoréfractomètre fournit des données qui sont ensuite utilisées pour piloter la tête de réfracteur automatique.

Certains appareils sont parfois équipés d'une petite imprimante thermique qui imprime les résultats de mesure sur un ticket, à charge au praticien de les reporter dans l'appareil suivant. On préfère toutefois que les appareils communiquent directement entre eux afin d'éviter au praticien une saisie fastidieuse et potentiellement source d'erreur. Certains fabricants prévoient ainsi que la console de commande de la tête de réfracteur automatique assure également le pilotage des appareils secondaires : projecteur ou écran, autoréfractomètre et/ou frontofocomètre. Cette console de commande offre alors une interface graphique parfois tactile avec des boutons de commande permettant de :
- changer les verres sur la tête de réfracteur automatique,
- choisir le test à afficher par le projecteur ou l'écran,
- recevoir des données provenant de l'autoréfractomètre et/ou du frontofocomètre,
- envoyer une mesure finale vers l'ordinateur.

Mais la console de commande est spécifiquement conçue pour fonctionner exclusivement avec certains appareils, par exemple en ce qui concerne les tests d'optotypes disponibles à la projection ou les plages de valeurs de verres disponibles sur la tête de réfracteur automatique, et incapable de fonctionner avec tout appareil tiers.

Actuellement, la communication entre les appareils, lorsqu'elle est possible, reste généralement limitée aux appareils d'un même fabricant, voire même d'une même gamme du fabricant. Les praticiens ne peuvent donc pas s'équiper chez différents fabricants sauf à renoncer à l'interopérabilité de leurs appareils. Renoncer à cette interopérabilité est dommage mais possible pour certains appareils comme l'autoréfractomètre ou le frontofocomètre. En revanche, le fonctionnement du projecteur ou écran d'optotypes est étroitement lié à celui de la tête de réfracteur automatique et il est difficile en pratique de se passer de leur synchronisation. Par ailleurs, il est également important pour éviter les erreurs de prescription et fluidifier les consultations que le résultat fourni par la tête de réfracteur automatique soit transmis automatiquement au logiciel patient à partir duquel sera imprimée l'ordonnance de prescription, mais tous les logiciels patients ne reconnaissent pas tous les types d'appareils. La situation actuelle présente donc de nombreux inconvénients :
- obligation de s'équiper exclusivement chez un fabricant ;
- en cas de panne d'un appareil et d'impossibilité de le remplacer à l'identique, par exemple parce que la gamme du fabricant a évolué, nécessité de devoir également remplacer d'autres appareils pourtant toujours fonctionnels ;
- coexistence d'interfaces utilisateurs variées, propres à chaque fabricant voire à chaque modèle d'appareil, ce qui requiert d'adapter les protocoles et former les personnels ;
- impossibilité de bénéficier d'outils d'aide au diagnostic, d'autodiagnostic ou de toute autre amélioration tierce en raison d'une conception propriétaire fermée ;
- frein à l'émergence de produits concurrentiels ne disposant pas d'une gamme complète.
Le document US 7730242 intitulé « Communication conversion system for switching first communication lines to second communication lines based on change in voltage state of detectionuse pin », le document US 2015/295764 intitulé « Methods and systems for secure interoperability between medical devices », le document US 2009/193435 intitulé « Converter for converting communication method and/or communication protocol » sont connus de l'état de la technique.

### RÉSUMÉ DE L'INVENTION

Un objectif de l'invention est de remédier à tout ou partie des inconvénients précités.

Un premier aspect de l'invention concerne un système de commande d'au moins un appareil d'examen de vue selon la revendication 1.

Dans la présente demande, on entend par « un premier élément connecté à un deuxième élément » le fait que les premier et deuxième éléments sont aptes à communiquer entre eux et utilisent donc un même protocole physique et un même langage logique. Dans la présente demande, on emploie indifféremment les termes « communication » et « communication bidirectionnelle ».

Le système de commande selon le premier aspect de l'invention permet ainsi d'assurer une interopérabilité entre des appareils d'examen de vue indépendamment de leur fabricant et/ou de leur modèle. Les praticiens, centres de santé, cliniques etc. peuvent ainsi choisir leurs appareils en fonction de leurs caractéristiques propres, par exemple de qualité, prix, disponibilité, performances etc., et non plus en fonction de leur fabricant ou modèle pour l'interopérabilité. Ils peuvent notamment remplacer un seul appareil défaillant de leur équipement, sans avoir à remplacer également d'autres appareils toujours fonctionnels.

Le système de commande selon le premier aspect de l'invention peut également présenter une ou plusieurs des caractéristiques ci-dessous, considérées individuellement ou selon toutes les combinaisons techniquement possibles :
- Le boîtier modulaire comporte une carte mère et, pour chaque appareil, une carte de gestion de protocole connectée à une carte de connexion ; la carte mère étant connectée à chaque carte de gestion de protocole ; chaque carte de connexion étant configurée pour être connectée à un appareil et utiliser le protocole physique dudit appareil ; chaque carte de gestion de protocole étant configurée pour assurer une communication entre sa carte de connexion qui utilise le protocole physique de l'appareil, et la carte mère qui utilise son propre protocole physique, en convertissant lesdits protocoles physiques.
- Le boîtier de pilotage peut être configuré pour utiliser le protocole physique de la carte mère. Dans ce cas, le boîtier de pilotage et la carte mère sont aptes à communiquer directement, sans l'intermédiaire d'une carte supplémentaire de gestion de protocole.
- Alternativement, le boîtier de pilotage peut être configuré pour utiliser son propre protocole physique, distinct du protocole physique de la carte mère. Dans ce cas, le boîtier modulaire comporte une carte supplémentaire de gestion de protocole connectée à la carte mère et au boîtier de pilotage et configurée pour assurer une communication entre la carte mère et le boîtier de pilotage en convertissant leurs protocoles physiques.
- Le boîtier de pilotage comporte une interface graphique et une interface technique ; l'interface graphique étant configurée pour recevoir des données d'entrée saisies par un utilisateur dans un langage logique humain, les convertir dans un langage logique métier et les transmettre sous cette forme à l'interface technique ; l'interface technique étant configurée pour recevoir des données dans le langage logique métier, les convertir dans le langage logique machine de l'appareil auquel elles sont destinées et les transmettre sous cette forme au boîtier modulaire.
- La carte mère du boîtier modulaire est connectée à l'interface technique du boîtier de pilotage, la carte mère et l'interface technique partageant un même langage logique machine et étant configurées pour communiquer des données dans tout langage logique machine d'un appareil en les encapsulant dans leur langage logique machine. Si la carte mère et le boîtier de pilotage partagent le même protocole physique, la carte mère est directement connectée à l'interface technique du boîtier de pilotage. Sinon, si la carte mère et le boîtier de pilotage ne partagent pas le même protocole physique, la carte mère est connectée à l'interface technique du boîtier de pilotage par l'intermédiaire de la carte supplémentaire de gestion de protocole du boîtier modulaire.
- L'encapsulation des données comporte avantageusement une information de provenance ou de destination d'un appareil.
- Au moins un appareil étant associé à un boîtier de contrôle, le boîtier modulaire est configuré pour être connecté avec chaque boîtier de contrôle et pour assurer une communication entre ledit au moins un appareil et son boîtier de contrôle dans le langage logique et selon le protocole physique dudit appareil.
- La carte de connexion dudit au moins un appareil est configurée pour être connectée audit au moins un appareil et au boîtier de contrôle dudit au moins un appareil.
- L'utilisateur est soit un humain, sur place ou à distance, soit un programme d'ordinateur.

Un deuxième aspect de l'invention concerne un procédé de commande d'au moins un appareil d'examen de vue selon la revendication 10.

Le procédé de commande selon le deuxième aspect de l'invention peut également comporter les étapes suivantes :
- le boîtier modulaire reçoit des données émises par un appareil et les transmet au boîtier de pilotage ;
- le boîtier de pilotage reçoit lesdites données et les interprète en fonction de l'appareil dont elles proviennent.

### BRÈVE DESCRIPTION DES FIGURES

D'autres caractéristiques et avantages de l'invention ressortiront clairement de la description qui en est donnée ci-dessous, à titre indicatif et nullement limitatif, en référence aux figures annexées, parmi lesquelles :
La figure 1 montre schématiquement un système de commande selon un premier mode de réalisation de l'invention, configuré pour fonctionner avec un premier appareil d'examen de vue.
La figure 2 montre schématiquement un système de commande selon un deuxième mode de réalisation de l'invention, configuré pour fonctionner avec des premier et deuxième appareils d'examen de vue.

Pour plus de clarté, les éléments identiques ou similaires sont repérés par des signes de référence identiques sur l'ensemble des figures.

### DESCRIPTION DÉTAILLÉE D'AU MOINS UN MODE DE RÉALISATION

Chaque type d'appareil d'examen de vue (autoréfractomètre, frontofocomètre, tête de réfracteur automatique etc.) et chaque modèle de type d'appareil, selon les fabricants voire selon les différentes gammes des fabricants, a son propre mode de fonctionnement à tous les niveaux :
- au niveau connectique, il existe une très grande diversité de types de prises, et une tendance des fabricants à ne pas respecter les normes et/ou usages. Une prise habituellement utilisée pour la vidéo des écrans d'ordinateur peut ainsi être utilisée pour la connectique d'une tête de réfracteur automatique ;
- plusieurs protocoles physiques de communication différents, de type bus ou point à point, sont susceptibles d'être utilisés : RS232, RS485, CanBus, etc. ;
- plusieurs langages logiques machines, propriétaires, sont également susceptibles d'être utilisés, qui déterminent le contenu des commandes à passer et le mode de communication entre chaque appareil et la console de commande (contrôle de flux, acquittement) ;
- enfin, les caractéristiques fonctionnelles peuvent tout à fait différer pour plusieurs appareils d'un même type : il peut s'agir par exemple des plages de verres disponibles et du pas de progression des verres pour une tête de réfracteur automatique ; ou des optotypes disponibles, des plages d'acuités disponibles pour un type d'optotype et du type d'optotype affiché à acuité donnée pour un projecteur.

D'une manière générale, le système de commande selon l'invention comporte un boîtier modulaire et un boîtier de pilotage. Le boîtier modulaire assure une adaptation connectique et de protocole physique : il prend en charge les différents types de prise et les différents protocoles physiques susceptibles d'être utilisés par les appareils d'examen de vue. Plus précisément, différentes cartes de connexion du boîtier modulaire prennent en charge différents types de prise, et différentes cartes de gestion protocole du boîtier modulaire prennent en charge différents protocoles physiques.

Le boîtier de pilotage assure quant à lui une adaptation de langage logique machine et de caractéristiques fonctionnelles : il prend en charge les différentes caractéristiques fonctionnelles des appareils et les différents langages logiques machine qu'ils sont susceptibles d'utiliser, ainsi que leur conversion dans un langage logique humain. Plus précisément, une interface technique du boîtier de pilotage prend en charge tous les langages logiques machines et assure leur conversion dans un langage logique métier compréhensible par une interface graphique du boîtier de pilotage, qui assure à son tour sa conversion dans un langage logique humain. L'interface graphique du boîtier de pilotage prend de son côté en charge les différentes caractéristiques fonctionnelles des appareils.

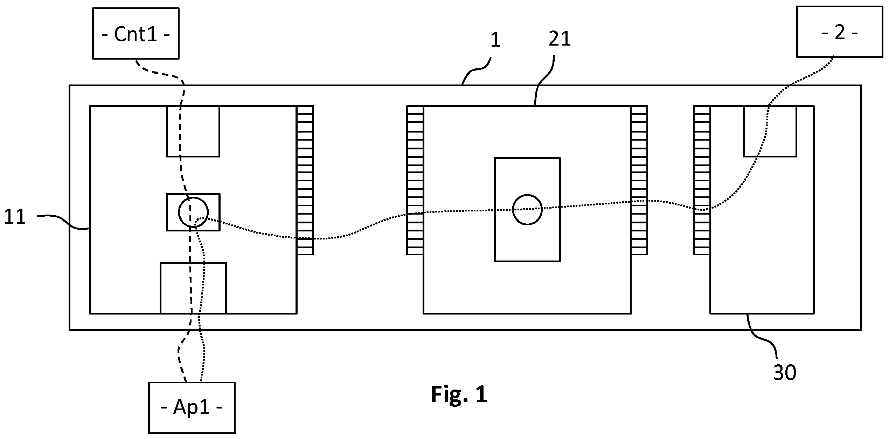

La figure 1 montre schématiquement un système de commande selon un premier mode de réalisation de l'invention, configuré pour commander un premier appareil Ap1 d'examen de vue, le système de commande comprenant un boîtier modulaire 1 et un boîtier de pilotage 2 connectés entre eux. Le premier appareil Ap1 communique dans un certain langage logique machine et selon un certain protocole physique. Le boîtier modulaire 1 est configuré pour être connecté avec le premier appareil Ap1 et pour assurer une communication entre le premier appareil Ap1, qui utilise son propre protocole physique, et le boîtier de pilotage 2 qui utilise son propre protocole physique, en convertissant lesdits protocoles physiques. Le boîtier de pilotage 2 est quant à lui configuré pour assurer une communication entre un utilisateur, qui utilise un langage logique humain et le premier appareil Ap1 qui utilise son propre langage machine, en convertissant lesdits langages logiques.

Le boîtier modulaire 1 comporte une carte mère 30, une première carte de gestion de protocole 21 et une première carte de connexion 11. La carte mère 30 est connectée à la première carte de gestion de protocole 21 et la première carte de gestion de protocole 21 est connectée à la première carte de connexion 11. La première carte de connexion 11 est configurée pour être connectée au premier appareil Ap1 et utiliser le protocole physique du premier appareil Ap1. La première carte de gestion de protocole 21 est configurée pour assurer une communication entre la première carte de connexion 11, qui utilise le protocole physique du premier appareil Ap1, et la carte mère 30 qui utilise son propre protocole physique, en convertissant lesdits protocoles physiques.

Le boîtier de pilotage 2 peut être configuré pour utiliser le protocole physique de la carte mère 30. Dans ce cas, le boîtier de pilotage 2 et la carte mère 30 sont aptes à communiquer directement, sans l'intermédiaire d'une carte supplémentaire de gestion de protocole.

Alternativement, le boîtier de pilotage 2 peut être configuré pour utiliser son propre protocole physique, distinct du protocole physique de la carte mère 30. Dans ce cas, le boîtier modulaire 1 comporte une carte supplémentaire de gestion de protocole connectée à la carte mère 30 et au boîtier de pilotage 2 et configurée pour assurer une communication entre la carte mère 30 et le boîtier de pilotage 2 en convertissant leurs protocoles physiques.

Le boîtier de pilotage 2 comporte une interface graphique et une interface technique. L'interface graphique est configurée pour recevoir des données d'entrée saisies par un utilisateur dans un langage logique humain, les convertir dans un langage logique métier et les transmettre sous cette forme à l'interface technique. L'interface technique quant à elle est configurée pour recevoir des données dans le langage logique métier, les convertir dans le langage logique du premier appareil Ap1 et les transmettre sous cette forme au boîtier modulaire 1.

La carte mère 30 du boîtier modulaire 1 est connectée à l'interface technique du boîtier de pilotage 2, la carte mère 30 et l'interface technique partageant un même langage logique machine et étant configurées pour communiquer des données dans tout langage logique machine, en particulier le langage logique machine du premier appareil Ap1, en les encapsulant dans leur langage logique machine partagé. L'encapsulation des données peut comporter une information de provenance ou de destination d'un appareil : information de provenance d'un appareil pour les données communiquées de la carte mère 30 au boîtier de pilotage 2 ; information de destination d'un appareil pour les données communiquées du boîtier de pilotage 2 à la carte mère 30.

Le premier appareil Ap1 est typiquement associé à un premier boîtier de contrôle Cnt1 et le boîtier modulaire 1 est avantageusement configuré pour être connecté au premier boîtier de contrôle Cnt1 et pour assurer une communication entre le premier appareil Ap1 et son premier boîtier de contrôle Cnt2 dans le langage logique et selon le protocole physique du premier appareil Ap1. La première carte de connexion 11 est alors configurée pour être connectée au premier appareil Ap1 et au premier boîtier de contrôle Cnt1.

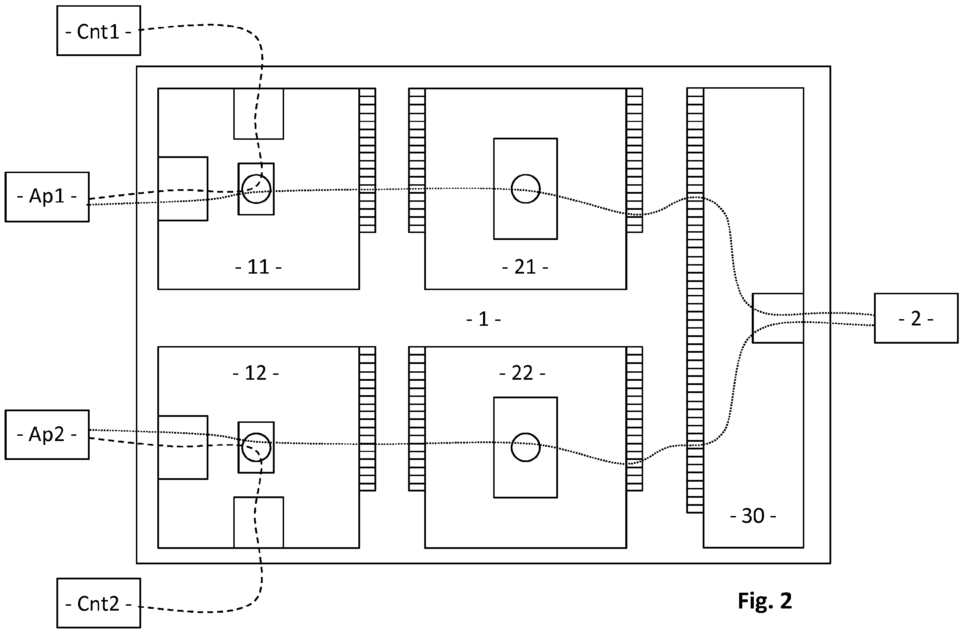

La figure 2 montre schématiquement un système de commande selon un deuxième mode de réalisation de l'invention, configuré pour commande le premier appareil Ap1 ainsi qu'un deuxième appareil Ap2 d'examen de vue. Sauf indication contraire par la suite, toutes les caractéristiques précédemment décrites du premier mode de réalisation s'appliquent au deuxième mode de réalisation. Chacun des premier et deuxième appareils Ap1, Ap2 communique dans un certain langage logique machine et selon un certain protocole physique. D'une manière générale, les premier et deuxième appareils Ap1, Ap2 peuvent utiliser le même langage logique machine ou bien deux langages logiques machines différents ; de même ils peuvent utiliser le même protocole physique ou bien deux protocoles physiques différents. En pratique, ils utilisent souvent le même protocole physique mais pas le même langage logique machine. Selon le deuxième mode de réalisation, le boîtier modulaire 1 est configuré pour être connecté avec les premier et deuxième appareils Ap1, Ap2 et pour assurer une communication entre chacun des premier et deuxième appareils Ap1, Ap2 et le boîtier de pilotage 2 ; le boîtier de pilotage 2 étant quant à lui configuré pour assurer une communication entre l'utilisateur et chacun des premier et deuxième appareils Ap1, Ap2. Le boîtier modulaire 1 assure désormais la conversion des protocoles physiques des premier et deuxième appareils Ap1, Ap2 et de la carte mère 30, ainsi que du boîtier de pilotage 2 s'il n'utilise pas le même protocole physique que la carte mère 30. De même, le boîtier de pilotage 2 assure désormais la conversion du langage logique humain et des langages logiques machines des premier et deuxième appareils Ap1, Ap2.

Le boîtier modulaire comporte la carte mère 30, la première carte de gestion de protocole 21 et une deuxième carte de gestion de protocole 22, la première carte de connexion 11 et une deuxième carte de connexion 12. La carte mère 30 est connectée aux première et deuxième cartes de gestion de protocole 21, 22. La première carte de gestion de protocole 21 est connectée à la première carte de connexion 11 ; et la deuxième carte de gestion de protocole 22 est connectée à la deuxième carte de connexion 12. La deuxième carte de connexion 12 est configurée pour être connectée au deuxième appareil Ap2 et utiliser le protocole physique du deuxième appareil Ap2. La deuxième carte de gestion de protocole 22 est configurée pour assurer une communication entre la deuxième carte de connexion 12, qui utilise le protocole physique du deuxième appareil Ap2, et la carte mère 30 qui utilise son propre protocole physique, en convertissant lesdits protocoles physiques.

L'interface technique du boîtier de pilotage 2 selon le deuxième mode de réalisation de l'invention est configurée pour recevoir de la part de l'interface graphique des données dans le langage logique métier, pour convertir ces données dans le langage logique machine de l'appareil, premier ou deuxième, auquel elles sont destinées, et pour les transmettre sous cette forme au boîtier modulaire 1.

Selon le deuxième mode de réalisation, la carte mère 30 et l'interface technique partagent un même langage logique machine et sont configurées pour communiquer des données dans tout langage logique machine, en particulier celui du premier appareil Ap1 et celui du deuxième appareil Ap2, en les encapsulant dans leur langage logique machine partagé. L'encapsulation des données comporte avantageusement une information de provenance ou de destination d'un appareil : information de provenance d'un appareil pour les données communiquées de la carte mère 30 au boîtier de pilotage 2 ; information de destination d'un appareil pour les données communiquées du boîtier de pilotage 2 à la carte mère 30.

Le deuxième appareil Ap2 est typiquement associé à un deuxième boîtier de contrôle Cnt2 et le boîtier modulaire est avantageusement configuré pour être connecté au deuxième boîtier de contrôle Cnt2 et pour assurer une communication entre le deuxième appareil Ap2 et son deuxième boîtier de contrôle Cnt2 dans le langage logique et selon le protocole physique du deuxième appareil Ap2. La deuxième carte de connexion 12 est alors configurée pour être connectée au deuxième appareil Ap2 et au deuxième boîtier de contrôle Cnt2. D'une manière générale, chaque appareil peut être associé à un boîtier de contrôle, ou bien seulement certains appareils peuvent être associés à un boîtier de contrôle.

Le système n'est pas limité à la commande de deux appareils d'examen de vue et pourrait en commander trois ou plus. Les appareils d'examen de vue sont par exemple un autoréfractomètre, un frontofocomètre, un ordinateur équipé d'un logiciel de gestion des patients, une tête de réfracteur automatique, un projecteur ou un écran d'optotypes.

Le système de commande selon l'invention est connecté à chaque appareil à piloter. Dans le cas où, selon l'art antérieur, une console de commande typiquement connectée au boîtier de contrôle de la tête de réfracteur automatique est déjà présente pour piloter certains appareils, le système de commande selon l'invention s'intercale entre le boîtier de contrôle et tous les appareils à piloter. Les appareils qui étaient déjà interconnectés par la console de commande le sont désormais par le système de commande, qui interconnecte en outre les appareils qui n'étaient pas pris en charge par la console de commande. Pour les appareils qui étaient déjà interconnectés par la console de commande, le système de commande selon l'invention maintient l'expérience utilisateur en se substituant à la console de commande via son interface graphique, qui reprend généralement les mêmes fonctionnalités que la console de commande. Pour les appareils qui n'étaient pas précédemment interconnectés par la console de commande, le système de commande selon l'invention améliore l'expérience utilisateur en permettant désormais leur pilotage via l'interface graphique du boîtier de pilotage. Le boîtier de pilotage se présente par exemple sous la forme d'une tablette ou d'une console.

Selon l'un quelconque des premier ou deuxième modes de réalisation, la carte mère 30 est par exemple un microcontrôleur d'usage général, comportant de préférence un port USB et plusieurs liaisons série, par exemple huit, et intégrant une mémoire de travail RAM, par exemple de 32 kb, et une mémoire Flash, par exemple de 256 kb pour stocker un programme applicatif et des paramètres de configuration. Le protocole physique de la carte mère 30 est par exemple le protocole TTL (de l'anglais « Transistor Transistor Logic »). Le protocole physique du boîtier de pilotage 2 est par exemple le protocole Bluetooth ou Wifi ou un protocole filaire et dans ce cas, le boîtier modulaire 1 comporte une carte supplémentaire de gestion de protocole assurant une conversion entre le protocole physique TTL de la carte mère 30 et le protocole Bluetooth, Wifi ou filaire du boîtier de pilotage 2. Le système de commande peut par exemple commander un ou plusieurs appareils d'examen de vue de la liste suivante :
- un autoréfractomètre, utilisant par exemple le protocole physique RS232 et étant connecté à une carte de connexion par exemple au moyen d'une prise DB9, la ou chaque carte de connexion étant connectée à une carte de gestion de protocole par exemple au moyen d'un relai commandé par GPIO, la carte de gestion de protocole assurant une conversion entre le protocole physique RS232 de l'autoréfractomètre et le protocole physique TTL de la carte mère 30 ;
- un frontofocomètre, utilisant par exemple le protocole physique RS232 et étant connecté à une carte de connexion par exemple au moyen d'une prise DB9, la ou chaque carte de connexion étant connectée à une carte de gestion de protocole par exemple au moyen d'un relai commandé par GPIO, la carte de gestion de protocole assurant une conversion entre le protocole physique RS232 du frontofocomètre et le protocole physique TTL de la carte mère 30 ;
- une tête de réfracteur automatique, utilisant par exemple le protocole physique RS232 ou RS485 et étant connecté à une carte de connexion par exemple au moyen d'une prise µSubD 20 ou Din8 ou DB15 ou DB9, la ou chaque carte de connexion étant connectée à une carte de gestion de protocole par exemple au moyen d'un relai commandé par GPIO, la carte de gestion de protocole assurant une conversion entre le protocole physique RS232 ou RS485 de la tête de réfracteur automatique et le protocole physique TTL de la carte mère 30 ;
- un projecteur, utilisant par exemple le protocole physique CAN ou RS232 et étant connecté à une carte de connexion par exemple au moyen d'une prise Din6 ou Din8 ou µSubD 32, la ou chaque carte de connexion étant connectée à une carte de gestion de protocole par exemple directement ou au moyen d'un relai commandé par GPIO, la carte de gestion de protocole assurant une conversion entre le protocole physique RS232 ou RS485 du projecteur et le protocole physique TTL de la carte mère 30 ;
- un ordinateur, utilisant par exemple le protocole physique RS232 et étant connecté à une carte de connexion par exemple au moyen d'une prise DB9, la ou chaque carte de connexion étant connectée à une carte de gestion de protocole par exemple au moyen d'un relai commandé par GPIO, la carte de gestion de protocole assurant une conversion entre le protocole physique RS232 de l'ordinateur et le protocole physique TTL de la carte mère 30.

Chaque appareil d'examen de vue peut généralement être connecté à un boîtier de contrôle, de manière filaire au moyen d'un câble de plusieurs conducteurs, ou de manière non-filaire. Dans l'exemple d'une connexion filaire, le câble comporte généralement deux conducteurs qui portent un signal utile et d'autres conducteurs qui portent une alimentation. Cette alimentation peut être nécessaire ou non à la communication.

La carte de connexion de l'appareil présente alors avantageusement à la fois une première prise adaptée à l'appareil, et une seconde prise adaptée au boîtier de contrôle de l'appareil. Cela permet à la carte de connexion d'assurer une continuité des conducteurs annexes (par exemple, les conducteurs responsables de l'alimentation) entre l'appareil et le boîtier de contrôle, donc de conserver des fonctions annexes (comme par exemple, l'alimentation), tout en prélevant le signal utile afin de le transmettre à la carte de gestion de protocole à laquelle elle est connectée.

Par ailleurs, chaque carte de connexion comporte de préférence une puce d'identification qui identifie l'appareil auquel correspond la carte de connexion. Chaque puce d'identification est fixée, par exemple soudée, à sa carte de connexion. En effet, une même prise peut être utilisée différemment d'un appareil à l'autre, selon son câblage interne : par exemple, pour une même prise DB9, le signal utile d'un premier appareil peut se trouver sur les broches (« pin ») 2 et 3 tandis que le signal utile d'un deuxième appareil se trouve sur les broches 7 et 9.

La carte de gestion de protocole reçoit le signal utile, dans le protocole physique de l'appareil dont il provient, par exemple RS232 ou RS485 ou CanBus, etc., c'est-à-dire avec certaines caractéristiques électriques (par exemple, +6V/-6V) et techniques de format, longueur de message etc., et le convertit dans le protocole physique de la carte mère, typiquement le protocole TTL nécessitant un signal série de 0 à +5V, afin de le transmettre à la carte mère 30. De même que pour les cartes de connexion, chaque carte de gestion de protocole comporte de préférence une puce d'identification qui identifie le type de protocole physique qu'elle gère. Toutes les cartes de gestion de protocole assurent une conversion avec le protocole physique de la carte mère 30.

La carte mère 30 reçoit le signal utile converti dans son protocole physique et l'encapsule dans son propre langage logique machine en lui ajoutant une information de provenance d'une carte de gestion de protocole et donc d'un appareil, avant de le transmettre à l'interface technique du boîtier de pilotage 2, éventuellement par l'intermédiaire de la carte de gestion de protocole supplémentaire dans le cas où le boîtier de pilotage 2 n'utilise pas le même protocole physique que la carte mère 30. La communication entre le boîtier modulaire 1 et le boîtier de pilotage 2 peut être filaire ou non-filaire, par exemple Bluetooth ou Wifi.

La carte mère 30 peut en outre assurer une ou plusieurs fonctions supplémentaires parmi les suivantes : authentification du boîtier de pilotage 2 avant toute transmission de signaux au boîtier de pilotage 2 ; découverte de la configuration lors d'une étape initiale décrite un peu plus loin ; paramétrage des cartes de gestion de protocole, en particulier de leur vitesse de transmission, à partir d'informations fournies par le boîtier de pilotage 2 une fois la configuration découverte.

Le boîtier modulaire 1 comporte une carte de connexion par type d'appareil d'examen de vue. Par exemple, cinq cartes de connexion : une première pour un autoréfractomètre, une deuxième pour un frontofocomètre, une troisième pour une tête de réfracteur automatique, une quatrième pour un projecteur ou écran et une cinquième pour un ordinateur. Le boîtier modulaire 1 pourrait comporter des cartes de connexion supplémentaires correspondant à d'autres types d'appareils. Le boîtier modulaire 1 peut également comporter moins de cinq cartes, par exemple seulement une, deux, trois ou quatre. Le boîtier modulaire est donc configuré différemment selon les appareils que l'on choisit d'y connecter par le biais des cartes de connexion. Il suffit de changer de carte de connexion pour changer d'appareil. Ainsi, chaque carte de connexion est amovible. De même, chaque carte de gestion de protocole est amovible. On entend par « une carte est amovible », que la carte peut être retirée du boîtier modulaire et ainsi être changée. Par exemple, si un appareil tombe en panne, il peut être remplacé par n'importe quel appareil du même type en changeant uniquement la carte de connexion et éventuellement la carte de gestion de protocole associée. Ainsi, le praticien peut modifier son panel d'appareils d'examen de vue sans perdre leur interopérabilité et n'est pas limité dans le choix de ces appareils. Chaque carte de connexion est associée à une carte de gestion de protocole. Le boîtier modulaire comporte un emplacement pour chaque binôme d'une carte de connexion et d'une carte de protocole. Les binômes sont de préférence toujours agencés dans le même ordre au sein du boîtier modulaire 1 : par exemple, le binôme du frontofocomètre dans le premier emplacement, le binôme de l'autoréfractomètre dans le deuxième emplacement, etc. Chaque carte de connexion comporte par ailleurs de préférence une puce d'identification du type de prise qu'elle gère, et chaque carte de gestion de protocole une puce d'identification du type de protocole physique qu'elle gère.

Le boîtier de pilotage 2 comporte par ailleurs une base de données qui fait correspondre à chaque appareil d'examen de vue son protocole physique, sa prise et son langage logique machine.

Ainsi, lors d'une étape initiale de configuration, le boîtier de pilotage 2 peut apprendre les appareils disponibles et la manière de communiquer avec eux : adresse de chaque appareil et langage logique machine parlé par chaque appareil. Pour ce faire, le boîtier de pilotage peut notamment envoyer un ou plusieurs signaux d'interrogation au boîtier modulaire 1, plus précisément à la carte mère 30 du boîtier modulaire 1. La carte mère 30 interroge alors successivement chaque emplacement du boîtier modulaire 1 pour apprendre les caractéristiques de chaque binôme carte de connexion/carte de gestion de protocole et en informer le boîtier de pilotage 2 au moyen d'un ou plusieurs signaux d'identification, chaque signal d'identification d'un appareil comportant une adresse, un type de carte de gestion de protocole et un type de carte de connexion. Au moyen de sa base de données et de chaque signal d'identification, le boîtier de pilotage 2 détecte les appareils pouvant être connectés et les propose automatiquement à l'utilisateur ou lui en demande confirmation, puis confirme le langage logique machine à utiliser, toujours au moyen de sa base de données.

Ainsi, quand une carte de connexion et/ou une carte de gestion de protocole est retirée et qu'une nouvelle carte de connexion et/ou une nouvelle carte de gestion de protocole est placée dans le boîtier modulaire 1 à la place de la carte retirée, l'étape initiale de configuration permet au boîtier de pilotage 2 d'apprendre les caractéristiques de la nouvelle carte de connexion et/ou la nouvelle carte de gestion de protocole et de pouvoir communiquer avec le nouvel appareil correspondant à la nouvelle carte de connexion et/ou la nouvelle carte de gestion de protocole.

L'utilisation d'un jeu de relais électromagnétiques commandés par GPIO permet, notamment dans le cas de liaisons RS232 ou RS485, de connecter chaque appareil au boîtier modulaire 1 lorsque ce dernier est sous tension, ou le cas échéant à son boîtier de contrôle lorsque le boîtier modulaire 1 est hors tension. Chaque carte de connexion comporte avantageusement, en plus d'une prise adaptée à son appareil, une prise adaptée à l'éventuel boîtier de contrôle de son appareil.

Le système de commande selon l'invention offre une interface graphique qui s'adapte précisément à chaque environnement (nombre, types, modèles d'appareils d'examen de vue, etc.) pour proposer des fonctionnalités compatibles avec les caractéristiques des appareils connectés et permet une commande centralisée de tous les appareils d'examen de vue d'un environnement donné. L'utilisateur ne voit donc pas, via l'interface graphique, des fonctionnalités standards dont certaines peuvent se révéler imprécises ou inaccessibles, mais bien les fonctionnalités exactes offertes par les appareils qu'il a connectés.

Cette interface graphique unique permet notamment l'installation d'outils d'aide au diagnostic, d'autodiagnostic, de télémédecine ou toute amélioration tierce. L'utilisateur du système de commande peut ainsi non seulement être un humain sur place, bénéficiant éventuellement d'un outil d'aide au diagnostic de manière à permettre une délégation de tâches, par exemple de l'ophtalmologue vers son assistant, mais également un humain à distance (télémédecine) ou encore un programme d'ordinateur (autodiagnostic).

Un procédé de commande d'au moins un appareil d'examen de vue par un utilisateur, au moyen du système de commande selon le premier ou deuxième mode de réalisation de l'invention, est à présent décrit.

Selon une première étape du procédé de commande, le boîtier de pilotage 2 reçoit des données saisies par l'utilisateur dans un langage logique humain et correspondant à une commande d'un appareil. Dans le premier mode de réalisation, cet appareil est le premier appareil Ap1 ; dans le deuxième mode de réalisation, cet appareil est soit le premier appareil Ap1, soit le deuxième appareil Ap2.

Selon une deuxième étape, le boîtier de pilotage 2 détermine l'appareil à commander, convertit les données dans le langage logique machine dudit appareil et transmet les données converties au boîtier modulaire 1 selon son protocole physique. La détermination par le boîtier de pilotage 2, à partir des données saisies par l'utilisateur dans son langage logique humain, de l'appareil à commander et du langage logique machine dans lequel convertir les données, se fait de la manière suivante :
- les données saisies par l'utilisateur correspondent à un type d'action propre à un type d'appareil (frontofocomètre, autoréfractomètre, tête de réfracteur automatique etc.). Par exemple, si l'utilisateur saisit une modification d'une caractéristique d'un verre (sphère, cylindre ou axe) de l'œil droit ou gauche, cela désigne une tête de réfracteur automatique. Si l'utilisateur saisit une modification d'une caractéristique d'un optotype (forme, taille, couleur...), cela désigne un écran ou projecteur. On détermine ainsi le type d'appareil à commander.
- Par ailleurs, grâce à l'étape initiale de configuration précédemment décrite, le boîtier de pilotage 2 connaît, pour chaque type possible d'appareil, le modèle particulier d'appareil disponible et la manière de communiquer avec lui : son adresse et le langage logique machine qu'il parle.

Selon une troisième étape, le boîtier modulaire 1 reçoit lesdites données selon le protocole physique du boîtier de pilotage, les convertit dans le protocole physique de l'appareil à commander et les transmet audit appareil.

Le procédé de commande peut en outre comporter une quatrième étape selon laquelle l'appareil à commander reçoit effectivement les données dans son langage logique machine et selon son protocole physique et réalise la commande initialement définie par l'utilisateur dans son langage logique humain via le boîtier de pilotage 2.

Le procédé de commande peut en outre comporter une cinquième étape selon laquelle le boîtier modulaire 1 reçoit des données émises par un appareil, les convertit dans le protocole physique du boîtier de pilotage et les transmet au boîtier de pilotage, ainsi qu'une sixième étape selon laquelle le boîtier de pilotage 2 reçoit lesdites données selon son protocole physique et les interprète en fonction de l'appareil dont elles proviennent. Le boîtier de pilotage peut avantageusement restituer les données interprétées à l'utilisateur, par exemple en les affichant sur l'interface graphique.

Il est fait référence aux différentes étapes du procédé de commande en les qualifiant de « première » étape, « deuxième » étape etc. mais cela ne signifie pas qu'elles doivent absolument se dérouler dans cet ordre. Par exemple, les cinquième et sixième étapes peuvent avoir lieu avant la quatrième étape.

## Revendications

1. Système de commande d'au moins un appareil (Ap1, Ap2) d'examen de vue, chaque appareil (Ap1, Ap2) communiquant dans un certain langage logique machine et selon un certain protocole physique, le système de commande comprenant :
- un boîtier de pilotage (2) configuré pour assurer une communication entre un utilisateur qui utilise un langage logique humain et chaque appareil (Ap1, Ap2) qui utilise son propre langage logique machine, en convertissant lesdits langages logiques ;
- un boîtier modulaire (1) connecté au boîtier de pilotage (2), le boîtier modulaire (1) étant configuré pour être connecté avec chaque appareil (Ap1, Ap2) et pour assurer une communication entre chaque appareil (Ap1, Ap2) qui utilise son propre protocole physique et le boîtier de pilotage (2) qui utilise son propre protocole physique, en convertissant lesdits protocoles physiques, le boîtier modulaire (1) comportant :
∘ pour chaque appareil (Ap1, Ap2), un emplacement accueillant un binôme constitué d'une carte de gestion de protocole (21, 22) et d'une carte de connexion (11, 12) connectées entre elles, chaque carte de connexion (11, 12) étant configurée pour être connectée à un appareil (Ap1, Ap2) et utiliser le protocole physique dudit appareil (Ap1, Ap2), chaque carte de connexion (11, 12) et chaque carte de gestion de protocole (21, 22) étant amovible ;
∘ une carte mère (30) connectée à chaque carte de gestion de protocole (21, 22), chaque carte de gestion de protocole (21, 22) étant configurée pour assurer une communication entre sa carte de connexion (11, 12) qui utilise le protocole physique de l'appareil (Ap1, Ap2), et la carte mère (30) qui utilise son propre protocole physique, en convertissant lesdits protocoles physiques.

2. Système de commande selon la revendication précédente, **caractérisé en ce que** le boîtier de pilotage (2) est configuré pour utiliser le protocole physique de la carte mère (30).

3. Système de commande selon l'une quelconque des revendications précédentes, **caractérisé en ce que** le boîtier de pilotage (2) est configuré pour utiliser son propre protocole physique, distinct du protocole physique de la carte mère (30), et **en ce que** le boîtier modulaire (1) comporte une carte supplémentaire de gestion de protocole connectée à la carte mère (30) et au boîtier de pilotage (2) et configurée pour assurer une communication entre la carte mère (30) et le boîtier de pilotage (2) en convertissant leurs protocoles physiques.

4. Système de commande selon l'une quelconque des revendications précédentes, **caractérisé en ce que** le boîtier de pilotage (2) comporte une interface graphique et une interface technique ; l'interface graphique étant configurée pour recevoir des données d'entrée saisies par un utilisateur dans un langage logique humain, les convertir dans un langage logique métier et les transmettre sous cette forme à l'interface technique ; l'interface technique étant configurée pour recevoir des données dans le langage logique métier, les convertir dans le langage logique machine de l'appareil (Ap1, Ap2) auquel elles sont destinées et les transmettre sous cette forme au boîtier modulaire (1).

5. Système de commande selon la revendication 4, **caractérisé en ce que** la carte mère (30) du boîtier modulaire (1) est connectée à l'interface technique du boîtier de pilotage (2), la carte mère (30) et l'interface technique partageant un même langage logique machine et étant configurées pour communiquer des données dans tout langage logique machine d'un appareil (Ap1, Ap2) en les encapsulant dans leur langage logique machine.

6. Système de commande selon la revendication précédente, **caractérisé en ce que** l'encapsulation des données comporte une information de provenance ou de destination d'un appareil (Ap1, Ap2).

7. Système de commande selon l'une quelconque des revendications précédentes dans lequel au moins un appareil (Ap1, Ap2) est associé à un boîtier de contrôle (Cnt1, Cnt2), **caractérisé en ce que** le boîtier modulaire (1) est configuré pour être connecté avec chaque boîtier de contrôle (Cnt1, Cnt2) et pour assurer une communication entre ledit au moins un appareil (Ap1, Ap2) et son boîtier de contrôle (Cnt1, Cnt2) dans le langage logique et selon le protocole physique dudit appareil (Ap1, Ap2).

8. Système de commande selon la revendication 7, **caractérisé en ce que** la carte de connexion (11, 12) dudit au moins un appareil (Ap1, Ap2) est configurée pour être connectée audit au moins un appareil (Ap1, Ap2) et au boîtier de contrôle (Cnt1, Cnt2) dudit au moins un appareil (Ap1, Ap2).

9. Système de commande selon l'une quelconque des revendications précédentes, **caractérisé en ce que** l'utilisateur est soit un humain, sur place ou à distance, soit un programme d'ordinateur.

10. Procédé de commande d'au moins un appareil (Ap1, Ap2) d'examen de vue par un utilisateur au moyen d'un système de commande selon l'une quelconque des revendications 1 à 9, comportant les étapes suivantes :
- le boîtier de pilotage (2) reçoit des données saisies par l'utilisateur dans un langage logique humain et correspondant à une commande d'un appareil (Ap1, Ap2) ;
- le boîtier de pilotage (2) détermine l'appareil (Ap1, Ap2) à commander, convertit les données dans le langage logique machine dudit appareil (Ap1, Ap2) et les transmet au boîtier modulaire (1) ;
- le boîtier modulaire (1) reçoit lesdites données, les convertit dans le protocole physique de l'appareil (Ap1, Ap2) et les transmet à l'appareil (Ap1, Ap2).

11. Procédé de commande selon la revendication précédente, **caractérisé en ce qu'**il comporte les étapes suivantes :
- le boîtier modulaire (1) reçoit des données émises par un appareil (Ap1, Ap2) et les transmet au boîtier de pilotage (2) ;
- le boîtier de pilotage (2) reçoit lesdites données et les interprète en fonction de l'appareil (Ap1, Ap2) dont elles proviennent.

12. Procédé de commande selon l'une quelconque des revendications 10 ou 11, **caractérisé en ce qu'**il comporte une étape initiale de configuration comportant les sous-étapes suivantes :
- le boîtier de pilotage (2) envoie un ou plusieurs signaux d'interrogation à la carte mère (30) ;
- la carte mère (30) interroge successivement chaque emplacement du boîtier modulaire (1) pour apprendre les caractéristiques de chaque binôme et informe le boîtier de pilotage (2) au moyen d'un ou plusieurs signaux d'identification, chaque signal d'identification comportant au moins une adresse, un type de carte de gestion de protocole et un type de carte de connexion.

## Patentansprüche

1. System zur Steuerung mindestens einer Vorrichtung (Ap1, Ap2) zur Durchführung eines Sehtests, wobei jede Vorrichtung (Ap1, Ap2) in einer bestimmten logischen Maschinensprache und gemäß einem bestimmten physikalischen Protokoll kommuniziert, wobei das Steuerungssystem folgendes umfasst:
- ein Steuerungsgehäuse (2), das konfiguriert ist, um eine Kommunikation zwischen einem Benutzer, der eine logische menschliche Sprache verwendet, und jeder Vorrichtung (Ap1, Ap2), die ihre eigene logische Maschinensprache verwendet, durch Konvertieren dieser logischen Sprachen zu gewährleisten;
- ein Modulgehäuse (1), das mit dem Steuerungsgehäuse (2) verbunden ist, wobei das Modulgehäuse (1) konfiguriert ist, um mit jeder Vorrichtung (Ap1, Ap2) verbunden zu werden und um eine Kommunikation zwischen jeder Vorrichtung (Ap1, Ap2), die ihr eigenes physikalisches Protokoll verwendet, und dem Steuerungsgehäuse (2), das sein eigenes physikalisches Protokoll verwendet, durch Konvertieren dieser physikalischen Protokolle zu gewährleisten, wobei das Modulgehäuse (1) folgendes umfasst:
o einen Steckplatz für jede Vorrichtung (Ap1, Ap2), wobei dieser jeweils ein Binom aufnimmt, das aus einer Protokoll-Steuerungsplatine (21, 22) und einer Anschlussplatine (11, 12) besteht, die miteinander verbunden sind, wobei jede Anschlussplatine (11, 12) konfiguriert ist, um mit einer Vorrichtung (Ap1, Ap2) verbunden zu werden und das physikalische Protokoll dieser Vorrichtung (Ap1, Ap2) zu verwenden, wobei jede Anschlussplatine (11, 12) und jede Protokoll-Steuerungsplatine (21, 22) herausnehmbar ist;
o eine Hauptplatine (30), die mit jeder Protokoll-Steuerungsplatine (21, 22) verbunden ist, wobei jede Protokoll-Steuerungsplatine (21, 22) konfiguriert ist, um eine Kommunikation zwischen ihrer Anschlussplatine (11, 12), die das physikalische Protokoll der Vorrichtung (Ap1, Ap2) verwendet, und der Hauptplatine (30), die ihr eigenes physikalisches Protokoll verwendet, durch Konvertieren dieser physikalischen Protokolle zu gewährleisten.

2. Steuerungssystem nach dem vorhergehenden Anspruch, **dadurch gekennzeichnet, dass** das Steuerungsgehäuse (2) konfiguriert ist, um das physikalische Protokoll der Hauptplatine (30) zu verwenden.

3. Steuerungssystem nach einem der vorhergehenden Ansprüche, **dadurch gekennzeichnet, dass** das Steuerungsgehäuse (2) konfiguriert ist, um sein eigenes physikalisches Protokoll zu verwenden, welches sich vom Protokoll der Hauptplatine (30) unterscheidet, und dass das Modulgehäuse (1) eine weitere Protokoll-Steuerungsplatine umfasst, die mit der Hauptplatine (30) und dem Steuerungsgehäuse (2) verbunden ist und konfiguriert ist, um eine Kommunikation zwischen der Hauptplatine (30) und dem Steuerungsgehäuse (2) durch Konvertieren ihrer physikalischen Protokolle zu gewährleisten.

4. Steuerungssystem nach einem der vorhergehenden Ansprüche, **dadurch gekennzeichnet, dass** das Steuerungsgehäuse (2) eine grafische Schnittstelle und eine technische Schnittstelle umfasst; wobei die grafische Schnittstelle konfiguriert ist, um Eingabedaten zu empfangen, die von einem Benutzer in einer logischen menschlichen Sprache eingegeben werden, diese in eine logische Fachsprache umzuwandeln und sie in dieser Form an die technische Schnittstelle zu übertragen; wobei die technische Schnittstelle konfiguriert ist, um Daten in der logischen Fachsprache zu empfangen, diese in die logische Maschinensprache der Vorrichtung (Ap1, Ap2) umzuwandeln, für die sie bestimmt sind, und sie in dieser Form an das Modulgehäuse (1) zu übertragen.

5. Steuerungssystem nach Anspruch 4, **dadurch gekennzeichnet, dass** die Hauptplatine (30) des Modulgehäuses (1) mit der technischen Schnittstelle des Steuerungsgehäuses (2) verbunden ist, wobei die Hauptplatine (30) und die technische Schnittstelle eine gemeinsame logische Maschinensprache haben und konfiguriert sind, um Daten in jeder logischen Maschinensprache einer Vorrichtung (Ap1, Ap2) zu kommunizieren, indem sie diese in ihre logische Maschinensprache kapseln.

6. Steuerungssystem nach dem vorhergehenden Anspruch, **dadurch gekennzeichnet, dass** die Datenkapselung eine Information von einer Vorrichtung (Ap1, Ap2) bezüglich der Herkunft oder des Ziels enthält.

7. Steuerungssystem nach einem der vorhergehenden Ansprüche, bei dem mindestens eine Vorrichtung (Ap1, Ap2) einem Kontrollgehäuse (Cnt1, Cnt2) zugeordnet ist, **dadurch gekennzeichnet, dass** das Modulgehäuse (1) konfiguriert ist, um mit jedem Kontrollgehäuse (Cnt1, Cnt2) verbunden zu werden und um eine Kommunikation zwischen dieser mindestens einen Vorrichtung (Ap1, Ap2) und ihrem Kontrollgehäuse (Cnt1, Cnt2) in der logischen Sprache und gemäß dem physikalischen Protokoll dieser Vorrichtung (Ap1, Ap2) zu gewährleisten.

8. Steuerungssystem nach Anspruch 7, **dadurch gekennzeichnet, dass** die Verbindungsplatine (11, 12) dieser mindestens einen Vorrichtung (Ap1, Ap2) konfiguriert ist, um mit dieser mindestens einen Vorrichtung (Ap1, Ap2) und dem Kontrollgehäuse (Cnt1, Cnt2) dieser mindestens einen Vorrichtung (Ap1, Ap2) verbunden zu werden.

9. Steuerungssystem nach einem der vorhergehenden Ansprüche, **dadurch gekennzeichnet, dass** der Benutzer entweder ein Mensch vor Ort oder remote ist oder ein Computerprogramm ist.

10. Verfahren zur Steuerung mindestens einer Vorrichtung (Ap1, Ap2) zur Durchführung eines Sehtests durch einen Benutzer mittels eines Steuerungssystems nach einem der Ansprüche 1 bis 9, das die folgenden Schritte umfasst:
- das Steuerungsgehäuse (2) empfängt Daten, die vom Benutzer in einer logischen menschlichen Sprache eingegeben werden und einer Steuerung einer Vorrichtung (Ap1, Ap2) entsprechen;
- das Steuerungsgehäuse (2) bestimmt die zu steuernde Vorrichtung (Ap1, Ap2), wandelt die Daten in die logische Maschinensprache dieser Vorrichtung (Ap1, Ap2) um und überträgt sie an das Modulgehäuse (1);
- das Modulgehäuse (1) empfängt diese Daten, wandelt sie in das physikalische Protokoll der Vorrichtung (Ap1, Ap2) um und überträgt sie an die Vorrichtung (Ap1, Ap2).

11. Steuerungsverfahren nach dem vorhergehenden Anspruch, **dadurch gekennzeichnet, dass** es die folgenden Schritte umfasst:
- das Modulgehäuse (1) empfängt Daten, die von einer Vorrichtung (Ap1, Ap2) gesendet werden und überträgt sie an das Steuerungsgehäuse (2);
- das Steuerungsgehäuse (2) empfängt diese Daten und interpretiert sie in Abhängigkeit von der Vorrichtung (Ap1, Ap2), von der sie stammen.

12. Steuerungsverfahren nach einem der Ansprüche 10 oder 11, **dadurch gekennzeichnet, dass** es einen anfänglichen Konfigurationsschritt mit den folgenden Unterschritten umfasst:
- das Steuerungsgehäuse (2) sendet ein oder mehrere Abfragesignale an die Hauptplatine (30);
- die Hauptplatine (30) fragt nacheinander jeden Steckplatz des Modulgehäuses (1) ab, um die Merkmale jedes Binoms in Erfahrung zu bringen und informiert das Steuerungsgehäuse (2) mittels eines oder mehrerer Identifikationssignale, wobei jedes Identifikationssignal mindestens eine Adresse, eine Typenbezeichnung für die Protokoll-Managementplatine und eine Typenbezeichnung für die Anschlussplatine umfasst.

## Claims

1. A system for controlling at least one vision test apparatus (Ap1, Ap2), each apparatus (Ap1, Ap2) communicating in a certain machine logic language and according to a certain physical protocol, the control system comprising:
- a drive box (2) configured to provide communication between a user which uses a human logic language and each apparatus (Ap1, Ap2) which uses its own machine logic language, by converting said logic languages;
- a modular box (1) connected to the drive box (2), the modular box (1) being configured to be connected with each apparatus (Ap1, Ap2) and to provide communication between each apparatus (Ap1, Ap2) which uses its own physical protocol and the drive box (2) which uses its own physical protocol, by converting said physical protocols, the modular box (1) including:
o for each apparatus (Ap1, Ap2), a location accommodating a binomial consisting of a protocol management board (21, 22) and a connection board (11, 12) connected to each other, each connection board (11, 12) being configured to be connected to an apparatus (Ap1, Ap2) and use the physical protocol of said apparatus (Ap1, Ap2), each connection board (11, 12) and each protocol management board (21, 22) being removable;
o a mother board (30) connected to each protocol management board (21, 22), each protocol management board (21, 22) being configured to provide communication between its connection board (11, 12) which uses the physical protocol of the apparatus (Ap1, Ap2), and the mother board (30) which uses its own physical protocol, by converting said physical protocols.

2. The control system according to the preceding claim, **characterised in that** the drive box (2) is configured to use the physical protocol of the mother board (30).

3. The control system according to any of the preceding claims, **characterised in that** the drive box (2) is configured to use its own physical protocol, distinct from the physical protocol of the mother board (30), and **in that** the modular box (1) includes an additional protocol management board connected to the mother board (30) and to the drive box (2) and configured to provide communication between the mother board (30) and the drive box (2) by converting their physical protocols.

4. The control system according to any of the preceding claims, **characterised in that** the drive box (2) includes a graphical interface and a technical interface; the graphical interface being configured to receive input data entered by a user in a human logic language, convert them in a business logic language and transmit them in this form to the technical interface; the technical interface being configured to receive data in the business logic language, convert them in the machine logic language of the apparatus (Ap1, Ap2) to which they are intended and transmit them in this form to the modular box (1).

5. The control system according to claim 4, **characterised in that** the mother board (30) of the modular box (1) is connected to the technical interface of the drive box (2), the mother board (30) and the technical interface sharing a same machine logic language and being configured to communicate data in any machine logic language of an apparatus (Ap1, Ap2) by encapsulating them in the machine logic language thereof.

6. The control system according to the preceding claim, **characterised in that** the encapsulation of data includes origin or destination information of an apparatus (Ap1, Ap2).

7. The control system according to any of the preceding claims, wherein at least one apparatus (Ap1, Ap2) is associated with a check box (Cnt1, Cnt2), **characterised in that** the modular box (1) is configured to be connected with each check box (Cnt1, Cnt2) and to provide communication between said at least one apparatus (Ap1, Ap2) and the check box (Cnt1, Cnt2) thereof in the logic language and according to the physical protocol of said apparatus (Ap1, Ap2).

8. The control system according to claim 7, **characterised in that** the connection board (11, 12) of said at least one apparatus (Ap1, Ap2) is configured to be connected to said at least one apparatus (Ap1, Ap2) and to the check box (Cnt1, Cnt2) of said at least one apparatus (Ap1, Ap2).

9. The control system according to any of the preceding claims, **characterised in that** the user is either a human, on the site or remote, or a computer program.

10. A method for controlling at least one vision test apparatus (Ap1, Ap2) by a user by means of a control system according to any of claims 1 to 9, including the following steps:
- the drive box (2) receives data entered by the user in a human logic language and corresponding to a command from an apparatus (Ap1, Ap2);
- the drive box (2) determines the apparatus (Ap1, Ap2) to be controlled, converts the data in the logic machine language of said apparatus (Ap1, Ap2) and transmits them to the modular box (1);
- the modular box (1) receives said data, converts them into the physical protocol of the apparatus (Ap1, Ap2) and transmits them to the apparatus (Ap1, Ap2).

11. The control method according to the preceding claim, **characterised in that** it includes the following steps:
- the modular box (1) receives data transmitted by an apparatus (Ap1, Ap2) and transmits them to the drive box (2);
- the drive box (2) receives said data and interprets them depending on the apparatus (Ap1, Ap2) from which they come.

12. The control method according to any of claims 10 or 11, **characterised in that** it includes an initial configuration step including the following sub-steps:
- the drive box (2) sends one or more query signals to the mother board (30);
- the mother board (30) successively queries each location of the modular box (1) to learn characteristics of each binomial and informs the drive box (2) by means of one or more identification signals, each identification signal including at least an address, a protocol management board type and a connection board type.
